# EUROPEAN PATENT SPECIFICATION

(11) **EP 3 054 925 B1**
(45) Date of publication and mention of the grant of the patent: **04.07.2018**
(21) Application number: 14799323.2
(22) Date of filing: 08.10.2014
(51) Int. Cl.: A61K 31/549, A61K 9/20, A61K 31/41, A61K 31/4422

(54) **A STABLE PHARMACEUTICAL COMPOSITION CONTAINING AMLODIPINE AND VALSARTAN**
FESTE DARREICHUNGSFORMEN ENTHALTEND VALSARTAN UND AMLODIPIN UND VERFAHREN ZU DEREN HERSTELLUNG.
COMPOSITION PHARMACEUTIQUE STABLE CONTENANT DE L'AMLODIPINE ET DU VALSARTAN

(30) Priority: 08.10.2013 CZ 20130783
(43) Date of publication of application: 17.08.2016
(73) Proprietor: Zentiva K.S., 102 37 Praha 10 (CZ)
(72) Inventor: SALANDOVA, Jana, 158 00 Praha 13 Stodulky (CZ); KRUMBHOLCOVA, Lucie, 276 01 Melnik (CZ); VARILOVA, Tereza, 142 00 Praha 4 (CZ); PRACNA, Marketa, 15800 Praha 13 Stodulky (CZ); KUKACKOVA, Lenka, 155 00 Praha 5 (CZ); BARTUNEK, Ales, 109 00 Praha 10 (CZ)
(74) Representative: Jirotkova, Ivana
(86) International application number: PCT/CZ2014/000113
(87) International publication number: WO 2015/051771

(56) References cited:
- WO-A1-03/097045
- WO-A1-2009/084003
- WO-A2-2007/022113

## Description

### Technical Field

The present invention relates to a stable pharmaceutical composition containing valsartan and amlodipine, which is in the form of a mono-layer tablet.

### Background Art

The patent application WO2000/02543A describes a combined therapy of hypertension with the AT1 receptor antagonist (S)-N-(1-carboxy-2-methylprop-1-yl)-N-pentanoyl-N-[2'(1H-tetrazol-5-yl)biphenyl-4-ylmethyl]amine (valsartan) or its pharmaceutically acceptable salt and the calcium channel blocker 3-ethyl-5-methyl(4RS)-2-[(2-aminoethoxy)methyl]-4-(2-chlorophenyl)-6-methyl-1,4-dihydropyridin-3,5-dicarboxylate (amlodipine) or its pharmaceutically acceptable salt.

A fixed combination containing valsartan and amlodipine in one dosage form is described in the patent application WO2007/022113A. The invention relates to a pharmaceutical composition in the form of a mono-layer tablet in the case of lower doses of valsartan (less than or equal to 160 mg) or bi-layer tablet in the case of higher doses of valsartan (more than 160 mg). The mono-layer tablets are prepared by dry granulation (compaction) of a mixture of amlodipine, valsartan and pharmaceutically acceptable excipients, followed by compression into tablets, which can be possibly coated. The bi-layer tablets are prepared by granulation of valsartan, dry or wet granulation of amlodipine; the obtained granules are subsequently individually compressed into a two-layered tablet, which can be possibly coated. Mono-layer tablets containing 320 mg of valsartan and 5 mg of amlodipine were also prepared in accordance with the invention, but in this case bioequivalence was not proved as compared to the mono components. Based on this finding, bi-layer tablets for higher strengths of valsartan (valsartan/amlodipine 320/5 mg and 320/10 mg) were developed.

The application WO2009/084003A describes a mono-layer tablet containing a combination of amlodipine and valsartan, which is bioequivalent as compared to the commercially available bi-layer tablet (EXFORGE ®), but the prepared tablets contain less than 35% by weight of valsartan and therefore tablets containing higher doses of valsartan are too big, which is inconvenient for the patient. The mono-layer tablets in accordance with the invention are prepared by dry granulation of both the active substances together with pharmaceutically acceptable excipients and subsequent compression of obtained granules into mono-layer tablets.

A pharmaceutical composition containing valsartan, amlodipine and hydrochlorothiazide is described in the application WO2003/097045A, where individual active substances are preferably separated from each other (kit of parts). The patent application WO2008002905A describes a pharmaceutical composition containing valsartan, amlodipine and hydrochlorothiazide in the form of mono-layer and bi-layer tablets. The mono-layer tablets are prepared by dry granulation (compaction) of a mixture of amlodipine, valsartan, hydrochlorothiazide and pharmaceutically acceptable excipients, the obtained granules are compressed into tablets, which can be possibly coated. It is known from the application WO20071022113A that dry granulation of active substances and pharmaceutically acceptable excipients does not allow to prepare a mono-layer tablet containing higher doses of valsartan (more than 160 mg) and amlodipine that is bioequivalent to the mono components. A similar problem is also expected in the case of a composition containing valsartan, amlodipine and hydrochlorothiazide, the design of which is not an object of the patent application WO20O8002905A.

It has been surprisingly found out that a mono-layer tablet containing a combination of amlodipine and valsartan can be prepared that is bioequivalent as compared to the mono components for all the commonly used doses (valsartan/amlodipine 160/5 mg, 160/10 mg, 320/5 mg and 320/10 mg), wherein the tablets of a higher strength have a lower weight compared to commercially available bi-layer tablets (EXFORGE ®), as well as to mono-layer tablets known from the prior art. It has been further found out that a mono-layer tablet containing a combination of amlodipine, valsartan and hydrochlorothiazide prepared by the method in accordance with the invention is also bioequivalent for all the commonly used doses.

### Disclosure of Invention

The invention provides a pharmaceutical composition, having the form of a mono-layer tablet, containing valsartan, amlodipine or their pharmaceutically acceptable salts and at least one pharmaceutically acceptable excipient. The pharmaceutical composition in accordance with the invention optionally contains hydrochlorothiazide as a further active ingredient.

In a preferable embodiment the pharmaceutical composition contains valsartan, amlodipine besylate and optionally hydrochlorothiazide as active ingredients. The amount of valsartan in a tablet is from 40 mg to 320 mg, preferably 80 mg, 160 mg and 320 mg; the amount of amlodipine is from 1.25 mg to 20 mg, preferably 2.5 mg, 5 mg and 10 mg; the amount of hydrochlorothiazide is from 5 mg to 50 mg, preferably 12.5 mg and 25 mg.

The amount of valsartan in a mono-layer tablet is more than 35% by weight, related to the tablet weight, and it is preferably in a micronized form to improve its biological availability. To ensure compaction of the fine material the wet granulation method has been selected.

In terms of formulation of amlodipine it was necessary to consider stability of the active ingredient itself on one hand and the critical parameter of content uniformity in the final dosage form on the other hand.

The pharmaceutical composition in accordance with the invention, having the form of a mono-layer tablet, contains pharmaceutically acceptable excipients selected from the group consisting of a filler, binder, lubricant, wetting agent, disintegrant and glidant.

Suitable fillers are microcrystalline cellulose, powdered cellulose, calcium hydrogen phosphate, calcium carbonate, silicified microcrystalline cellulose, lactose monohydrate, anhydrous lactose, mannitol, sorbitol, lactitol, fructose, dextrans, sucrose, magnesium carbonate, starch, pre-gelatinized starch.

Suitable binders are hydroxypropyl cellulose, hydroxyethyl cellulose, starch, pre-gelatinized starch, polymethacrylates, gelatine, hypromellose, povidone and microcrystalline cellulose.

Suitable disintegrants are crospovidone, croscarmellose sodium, starch, pre-gelatinized starch, microcrystalline cellulose, hydroxypropyl cellulose, sodium carboxymethyl starch, polacrilin potassium.

Suitable wetting agents are sodium lauryl sulfate, polysorbates.

Suitable glidants are talc, starch, colloidal silicon dioxide.

Suitable anti-adhesives (lubricants) are magnesium stearate, stearic acid, magnesium silicate, calcium stearate, sodium stearyl fumarate, macrogols, hydrogenated vegetable oils, sodium lauryl sulfate.

The pharmaceutical agent in accordance with the invention contains:

| | |
|---|---|
| Valsartan | 35 - 45 % by weight |
| Amlodipine | 0.5 - 4 % by weight |
| Filler | 32 - 54.5% by weight |
| Anti-adhesive agent | 0.2 - 2 % by weight |
| Glidant | 0.1 - 1 % by weight |
| Binder | 2 - 6 % by weight |
| Disintegrant | 1 - 8 % by weight |
| Wetting agent | 0.2 - 2 % by weight |

The mono-layer tablet in accordance with the invention is optionally provided with a coating.

The monolayer tablets in a preferred embodiment contain fillers in the range of 32 to 54.5% by weight, such as microcrystalline cellulose, powdered cellulose, calcium hydrogen phosphate, calcium carbonate, silicified microcrystalline cellulose, lactose monohydrate, anhydrous lactose, mannitol, sorbitol, lactitol, fructose, dextrans, sucrose, magnesium carbonate, starch, pre-gelatinized starch. From the group of anti-adhesives (lubricants), used in the range of 0.2 to 2% by, weight it can be magnesium stearate, stearic acid, magnesium silicate, calcium stearate, sodium stearyl fumarate, macrogols, hydrogenated vegetable oils, sodium lauryl sulfate. Glidants, used in the range of 0.1 to 1% by weight, are represented by talc, starch and colloidal silicon dioxide. Binders, present in amounts of 2 to 6% by weight, are hydroxypropyl cellulose, hydroxy ethyl cellulose, starch, pre-gelatinized starch, polymethacrylates, gelatine, hypromellose, povidone and microcrystalline cellulose. A disintegrant is selected from the group containing crospovidone, croscarmellose sodium, starch, pre-gelatinized starch, microcrystalline cellulose, hydroxypropyl cellulose, sodium carboxymethyl starch, polacrilin potassium, usually used in amounts of 1 to 8% by weight.

The invention further relates to a production method of a mono-layer tablet containing amlodipine and valsartan.

Valsartan, together with at least one pharmaceutically acceptable excipient, is granulated by kneading in a granulation device (high shear mixer), or by means of fluidized air (fluidized bed granulator), water being used as a wetting agent On achievement of the desired granules the mixture is dried to the resulting granulate humidity of 1 to 3% by weight.

Amlodipine besylate, together with at least one pharmaceutically acceptable excipient, is dry granulated, without the use of a wetting agent - i.e. granules are produced either by briquetting, being compressed to solid pieces that are subsequently crushed and sieved to powder. Another option is the compaction method, where a mixture of the active substance and excipient is compressed between two rollers that rotate in opposite directions and the mixture is compacted by pressure, and subsequently sieved. A force of 3 to 12 kN/cm is used for the compaction.

The compaction product of amlodipine is mixed with granules of valsartan and at least one pharmaceutically acceptable excipient, the mixture is homogenized and the tableting blend is compressed to solid pieces - while the obtained cores have the minimum strength of 30 N. After the compression coating is applied on the cores and the tablets are subjected to final drying.

The invention further relates to a production method of a mono-layer tablet containing amlodipine, valsartan and hydrochlorothiazide.

Valsartan, together with at least one pharmaceutically acceptable excipient, is granulated by kneading in a granulation device (high shear mixer), or by means of fluidized air (fluidized bed granulator), water being used as a wetting agent. On achievement of the desired granules the mixture is dried to the resulting granulate humidity of 1 to 3%.

Amlodipine besylate, together with at least one pharmaceutically acceptable excipient, is dry granulated, without the use of a wetting agent - i.e. granules are produced either by briquetting, being compressed to solid pieces that are subsequently crushed and sieved to powder. Another option is the compaction method, where a mixture of the active substance and excipient is compressed between two rollers that rotate in opposite directions and the mixture is compacted by pressure, and subsequently sieved. A force of 3 to 12 kN/cm is used for the compaction.

The compaction product of amlodipine is mixed with granules of valsartan and hydrochlorothiazide itself, with at least one pharmaceutically acceptable excipient, the mixture is homogenized and the tableting blend is compressed to solid pieces - while cores with the minimum strength of 30 N are obtained. After the compression coating is applied on the cores and the tablets are subjected to final drying.

Advantages of the formulation in accordance with the invention clearly include a higher stability resulting from the use of the dry processing method for amlodipine besylate and acceptable values of content uniformity. All dosage forms are produced by the same technology. The achieved stability has confirmed that the selected production technology ensures compatibility of both the active substances. The dosage form is stable even in spite of being a mono-layer tablet where both the active substances get more in contact. The production of mono-layer tablets does not require the use of any special tableting equipment; the production is feasible in conventional tableting machines. Further, the tablet size is very advantageous, especially in terms of patient's compliance. A mono-layer tablet in accordance with the invention containing the maximum used amount of the active substances, i.e. 10 mg of amlodipine and 320 mg of valsartan, only weighs 768 mg, while the weight of the original preparation Exforge® is more than 900 mg.

### Brief Description of Drawings

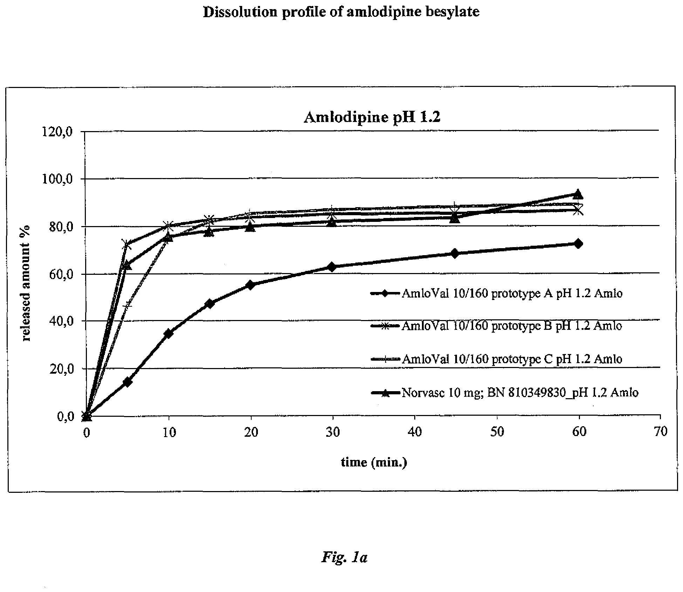

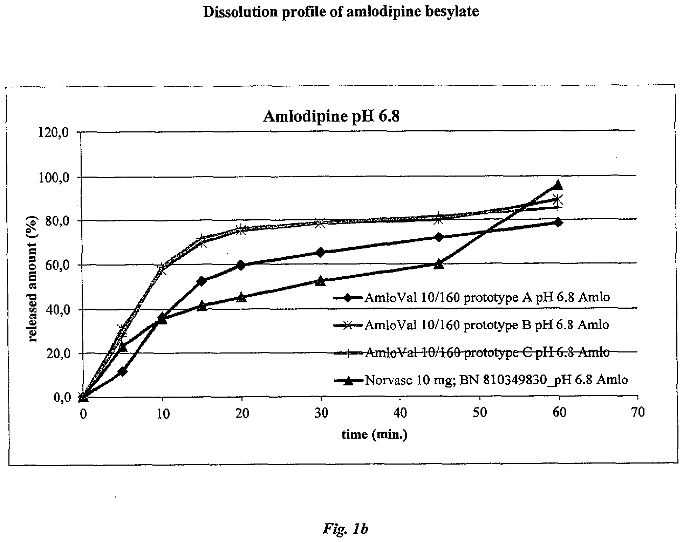

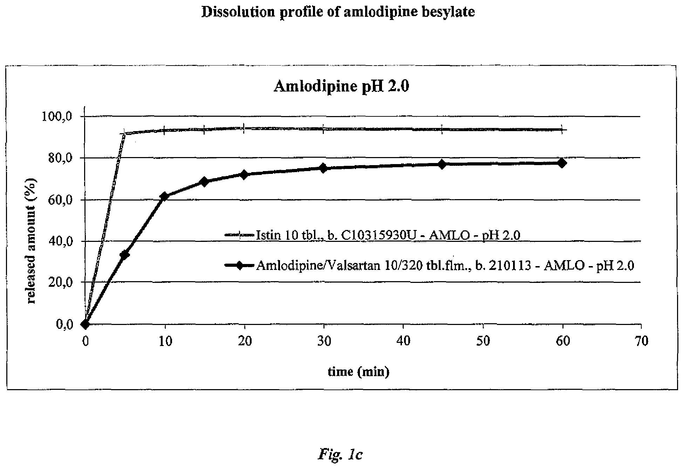

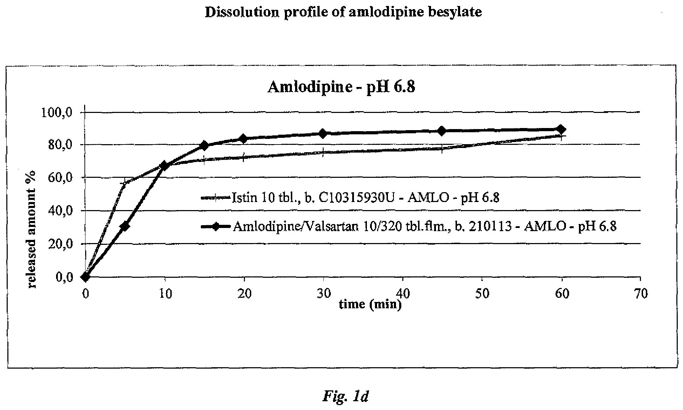

***Fig. 1*****: Dissolution profiles of amlodipine besylate**
   The figures shows the dissolution profiles of three prototypes of amlodipine/valsartan 10/160 mg compared to the original preparation Norvasc® (another name Istin®). Prototype A is slower in both the environments of pH 1.2 and pH 6.8 - amlodipine besylate is released more slowly from the granules. This means that from the dissolution point of view prototypes B and C appear to be suitable. Figures 1c and 1d compare the higher strength of amlodipine/valsartan compared to the original amlodipine Istin®.
***Fig. 2*****: Dissolution profiles of valsartan**
   The figures show the same trend, i.e. the slowest dissolution profile for prototype B of amlodipine/valsartan 10/160. The profiles are compared to the original valsartan product - Diovan®. Comparison of the higher strength of 10/320 mg is shown in Figs. 2c and 2d.
***Fig. 3*****: Dissolution profile of valsartan after 3M stability testing at pH 6.8**
   All the three developed prototypes A, B and C were loaded for 3 months in the conditions of 40°C and 75% relative humidity. The dissolution profiles got considerably decelerated for prototypes A and C. The figure shows profiles for valsartan, which are more critical in terms of bioequivalence. Prototype B exhibited the most stable formulation.

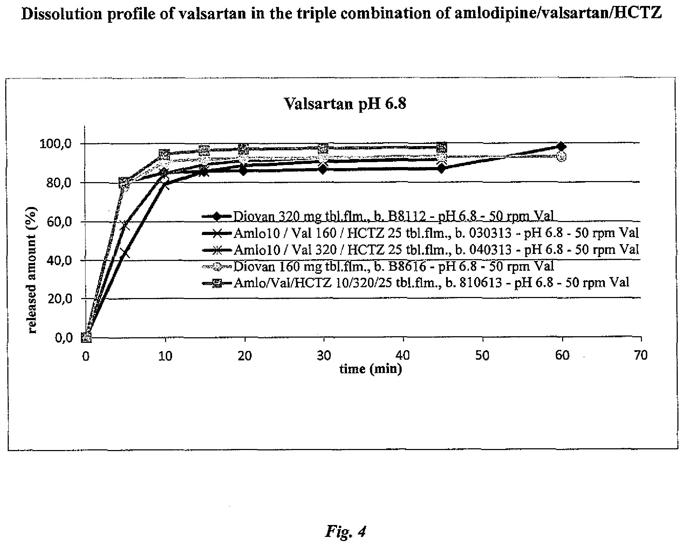

***Fig. 4*****: Dissolution profile of valsartan in the triple combination of amlodipine/valsartan/HCTZ**
   Figure 4 describes the dissolution profile of valsartan in the triple combination with amlodipine and hydrochlorothiazide. The releasing exhibits the same trend as in the case of the double combination and therefore in the case of this prototype bioequivalence comparable to the original preparation Diovan® is expected to be proved.

### Examples

**Example 1** - a mono-layer tablet made of a granulate containing valsartan (wet granulation) and powdered amlodipine. The values are quoted in mg.

**Table 1**

| **Composition of 1 tbl.flm.** | **Prototype 1** |
|---|---|
| Valsartan | 160.0 |
| Amlodipine besylate | 13.9 |
| Silicified microcrystalline cellulose | 67.0 |
| Sorbitol | 18.5 |
| Calcium carbonate + pre-gelatinized starch | 18.5 |
| Pre-gelatinized starch | 6.0 |
| Povidone | 15.0 |
| Sodium stearyl fumarate | 8.0 |
| Sodium lauryl sulphate | 2.0 |
| Crospovidone | 26.0 |
| Colloidal silicon dioxide | 4.0 |
| Coating layer (Macrogol, hypromellose, titanium dioxide, talc, iron oxides) | 10.0 |
| ***Total*** | ***348.9*** |

Valsartan, together with sorbitol, crospovidone, silicified microcrystalline cellulose, povidone and sodium lauryl sulfate is homogenized in a granulator and subsequently granulated by kneading with the use of water as a wetting agent. On achievement of the desired granules the mixture is dried to the resulting granulate humidity of 1 to 3% by weight. Amlodipine besylate is added to the granulate together with the excipients - the other part of crospovidone, pre-gelatinized starch, calcium carbonate with microcrystalline cellulose, sodium stearyl fumarate and colloidal silicon dioxide sieved through a sieve with the mesh size of max. 1.0 mm. After final homogenization the tableting blend is compressed to solid pieces with a strength of at least 30 N. After the compression the cores are coated with a hypromellose suspension. When the weight of the cores has increased by 10 mg, the coating is completed and the tablets are subjected to final drying. The temperature of the product should not exceed 42°C.
Tablets produced this way had a lower content of amlodipine; repeated experiments proved the same results. Admixing of amlodipine in the extragranular phase is critical; therefore other production methods have been tested, which are described below.

**Example 2** - a mono-layer tablet made of a granulate containing valsartan together with amlodipine (wet granulation). The values are quoted in mg.

**Table 2**

| **Composition of 1 tbl.flm.** | **Prototype A** |
|---|---|
| Valsartan | 160.0 |
| Amlodipine besylate | 13.9 |
| Silicified microcrystalline cellulose | 67.0 |
| Sorbitol | 18.5 |
| Calcium carbonate + pre-gelatinized starch | 18.5 |
| Pre-gelatinized starch | 6.0 |
| Povidone | 15.0 |
| Sodium stearyl fumarate | 8.0 |
| Sodium lauryl sulphate | 2.0 |
| Crospovidone | 26.0 |
| Colloidal silicon dioxide | 4.0 |
| Coating layer (Macrogol, hypromellose, titanium dioxide, talc, iron oxides) | 10.0 |
| ***Total*** | ***348.9*** |

Valsartan, together with amlodipine, sorbitol, crospovidone, silicified microcrystalline cellulose, povidone and sodium lauryl sulfate, is homogenized in a granulator and subsequently granulated by kneading with the use of water as a wetting agent. On achievement of the desired granules the mixture is dried to the resulting granulate humidity of 1 to 3%. Other excipients are added to the granulate - the other part of crospovidone, pre-gelatinized starch, calcium carbonate with microcrystalline cellulose, sodium stearyl fumarate and colloidal silicon dioxide sieved through a sieve with the mesh size of max. 1.0 mm. After final homogenization the tableting blend is compressed to solid pieces with a strength of at least 30 N. After the compression the cores are coated with a hypromellose suspension. When the weight of the cores has increased by 10 mg, the coating is completed and the tablets are subjected to final drying. The temperature of the product should not exceed 42°C.

**Example 3** - a mono-layer tablet made of a granulate containing valsartan (wet granulation) and compacted amlodipine.

**Table 3**

| **Composition of 1 tbl.flm.** | **Prototype B (mg)** |
|---|---|
| Valsartan | 160.0 |
| Amlodipine besylate | 13.9 |
| Silicified microcrystalline cellulose | 67.0 |
| Sorbitol | 18.5 |
| Calcium carbonate + pre-gelatinized starch | 18.5 |
| Pre-gelatinized starch | 6.0 |
| Povidone | 15.0 |
| Sodium stearyl fumarate | 9,0 |
| Sodium lauryl sulphate | 2.0 |
| Crospovidone | 36.0 |
| Colloidal silicon dioxide | 4.8 |
| Microcrystalline cellulose | 74.3 |
| Coating layer (Macrogol, hypromellose, titanium dioxide, talc, iron | 10.0 |
| ***Total*** | ***435.0*** |

Valsartan, together with sorbitol, crospovidone, silicified microcrystalline cellulose, povidone and sodium lauryl sulfate is homogenized in a granulator and subsequently granulated by kneading with the use of water as a wetting agent. On achievement of the desired granules the mixture is dried to the resulting granulate humidity of 1 to 3% by weight. Amlodipine besylate, together with microcrystalline cellulose, crospovidone, colloidal silicon dioxide and sodium stearyl fumarate is screened through a sieve with the mesh size of 1.0 mm and the mixture is compacted in a compactor. The compacted material is admixed to the granulate together with the remaining excipients - remaining part of crospovidone, pre-gelatinized starch, calcium carbonate with microcrystalline cellulose, sodium stearyl fumarate and colloidal silicon dioxide. After final homogenization the tableting blend is compressed to solid pieces with a strength of at least 30 N. After the compression the cores are coated with a hypromellose suspension. When the weight of the cores has increased by 10 mg, the coating is completed and the tablets are subjected to final drying. The temperature of the product should not exceed 42°C.

**Example 4** - a mono-layer tablet made of a granulate containing valsartan (wet granulation) and a granulate containing amlodipine (wet granulation).

**Table 4**

| **Composition of 1 tbl.flm.** | **Prototype C** |
|---|---|
| Valsartan | 160.0 |
| Amlodipine besylate | 13.9 |
| Silicified microcrystalline cellulose | 67.0 |
| Sorbitol | 18.5 |
| Calcium carbonate + pre-gelatinized starch | 18.5 |
| Pre-gelatinized starch | 6.0 |
| Povidone | 19,5 |
| Sodium stearyl fumarate | 8.0 |
| Sodium lauryl sulphate | 2.0 |
| Crospovidone | 26.0 |
| Colloidal silicon dioxide | 4.0 |
| Lactose monohydrate | 34.8 |
| Starch | 23.5 |
| Coating layer (Macrogol, hypromellose, titanium dioxide, talc, iron | 10.0 |
| ***Total*** | ***411.7*** |

Valsartan, together with sorbitol, crospovidone, silicified microcrystalline cellulose, povidone and sodium lauryl sulfate is homogenized in a granulator and subsequently granulated by kneading with the use of water as a wetting agent. On achievement of the desired granules the mixture is dried to the resulting granulate humidity of 1 to 3% by weight. Amlodipine besylate is granulated together with lactose, starch and povidone by kneading with water. The granulate is then dried to achieve the humidity of 1 to 3% by weight. The two granulates are mixed and finally homogenized with the remaining excipients. The tableting blend is compressed to solid pieces with a strength of at least 30 N. After the compression the cores are coated with a hypromellose suspension. When the weight of the cores has increased by 10 mg, the coating is completed and the tablets are subjected to final drying. The temperature of the product should not exceed 42°C.

### Comparison of the prototypes

All the three prototypes A, B and C were compared to each other. The tablets were loaded with accelerated conditions - temperature of 40°C and relative humidity of 75%. Critical parameters were monitored, especially purity, content uniformity and dissolution. The results of the tests are shown in Table 5.

**Table 5: Analytical evaluation of the prototypes**

| | | **Prototype A** | **Prototype B** | **Prototype C** |
|---|---|---|---|---|
| **Content of amlodipine** | start | 9.9 mg | 9.9 mg | 9.9 mg |
| | 3M 40°C 75% RH | 9.7 mg | 9.8 mg | 10.0 mg |
| | CU | 5.4 | 6.0 | 9.0 |
| **Content of valsartan** | start | 157.4 mg | 155.6 mg | 154.5 mg |
| | 3M 40°C 75% RH | 157.7 mg | 154.5 mg | 155.5 mg |
| | CU | 5.9 | 3.9 | 4.8 |
| **Purity** | start | 0.20% by weight Imp D (A) <0.05% by weight Imp B (V) | 0.25% by weight Imp D (A) <0.05% by weight Imp B (V) | 0.16% by weight Imp E (A) <0.05% by weight Imp B (V) |
| | 3M40°C 75% RH | 0.86% by weight Imp D (A) <0.05% by weight Imp B (V) | 0.37% by weight Imp D (A) <0.05% by weight Imp B (V) | 0.53% by weight Imp F (A) <0.05% by weight Imp B (V) |
| **Water** | start | 2.2% by weight | 2.6% by weight | 2.6% by weight |
| | 3M 40°C 75% RH | 5.9% by weight | 6.2% by weight | 6.2% by weight |

| | | | | |
|---|---|---|---|---|
| RH - relative humidity M - month CU - content uniformity Imp B (V) - impurity B (Valsartan)- *benzyl(2S)-3-methyl-2-[(pentanoyl[[(2'-(1H-tetrazol-5-yl) biphenyl*-*4-yl)methyl)amino)butanoate* Imp D (A) - impurity D (Amlodipine)-*3-ethyl5-methyl2-[(2-aminoethoxy)methyl]-4-(2-chlorophenyl)-6-methylpyridine-3,5-dicarboxylate* Imp E (A) - impurity E (Amlodipine)-*diethyl(4RS)-2-[(2-aminoethoxy)methyl]-4-(2-chlorophenyl)-6-methyl-1,4-dihydropyridine-3, 5-dicarboxylate* Imp F (A) - impurity F (Amlodipine)-*dimethyl(4RS)-2-[(2-aminoethoxy)methyl]-4-(2-chlorophenyl)-6-methyl-1,4-dihydropyridine-3,5-dicarboxylate* | | | | |

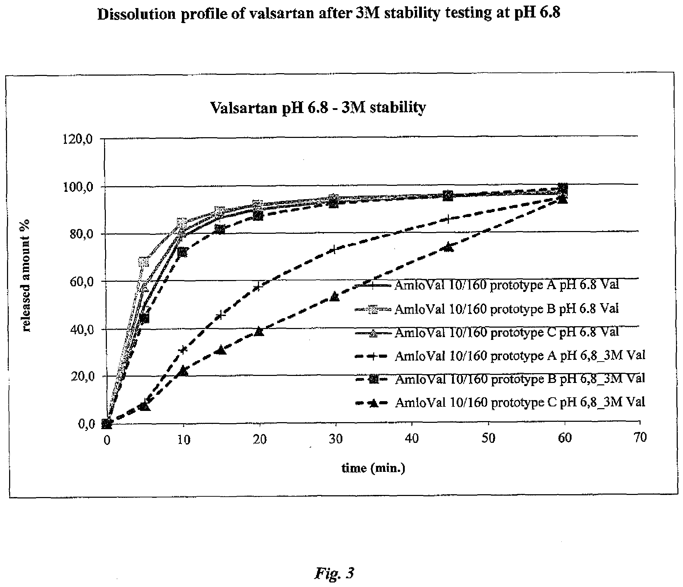

The content and content uniformity are satisfactory for all the prototypes. The change of the preparation method of amlodipine as compared to direct mixing (Example 1) has brought a solution to the problem of content non-uniformity. However, there are perceivable differences between the prototypes in terms of purity. The worst case is prototype A, where both the active substances are granulated together in one step. After three months under the accelerated conditions the content of impurity D increases up to the value of 0.86% by weight. From the purity point of view it is prototype B, where amlodipine besylate is processed using a dry method - compaction, that appears to be the best. Prototype A is not acceptable from the dissolution point of view either. The dissolution profile for amlodipine is considerably slower. In addition, the dissolution profile of valsartan is more critical. It is prototype B that exhibited the best results again. In the case of amlodipine granulation, the production method used for prototypes A and C, the profile of valsartan got considerably slower during the stability tests (Figure No. 3).

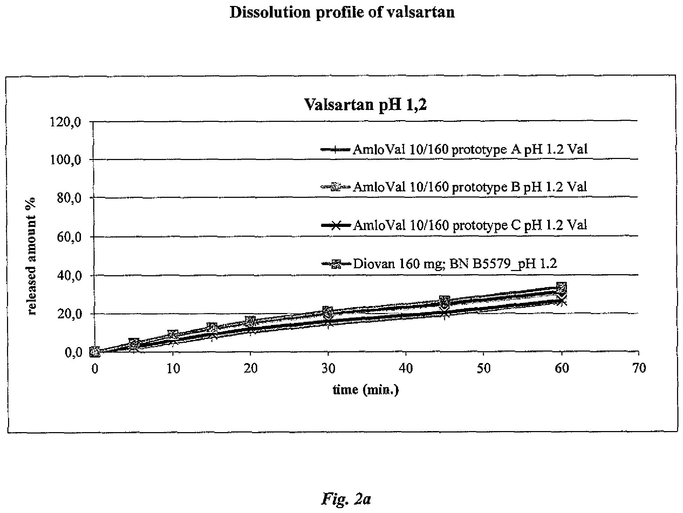

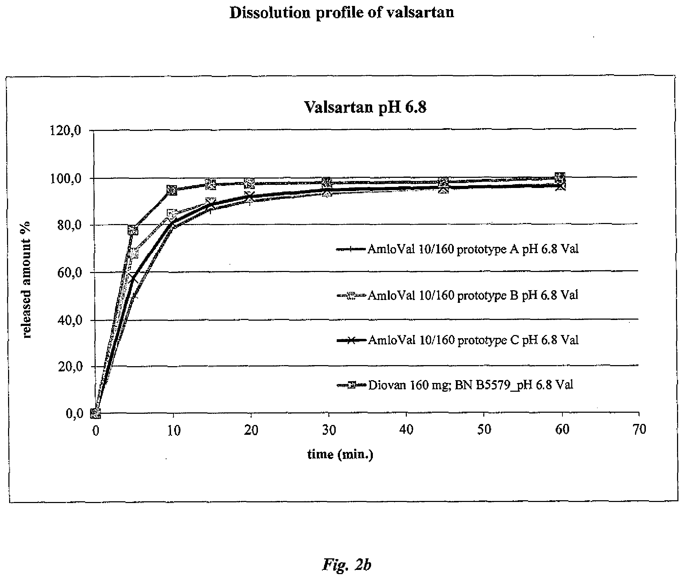

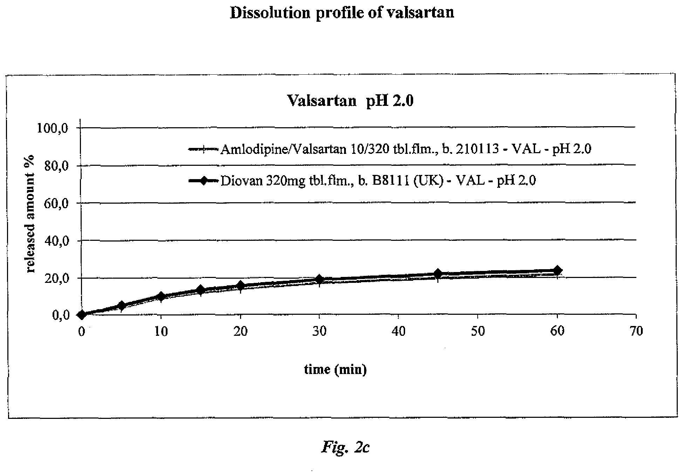

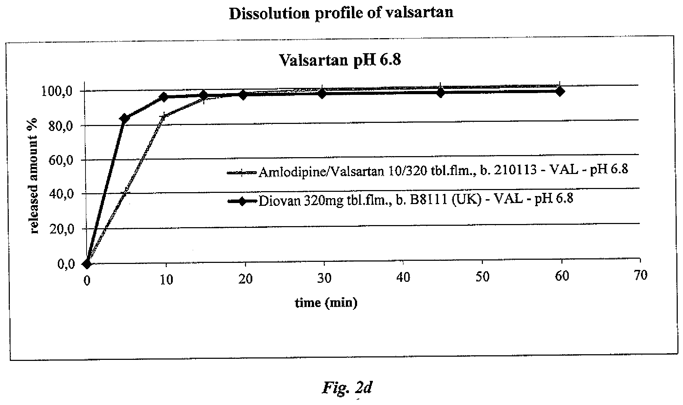

According to the results presented in Table 5 prototype B is the most stable one (Example 3, in which the granulate of amlodipine besylate (prepared by dry granulation - compaction) is admixed to the granulate of valsartan (prepared by wet granulation) together with extragranular excipients; after subsequent homogenization the tableting blend is compressed to solid pieces - cores, which are provided with a coating. In a similar way mono-layer tablets containing 320 mg of valsartan and 5 mg of amlodipine and 320 mg of valsartan and 10 mg of amlodipine were prepared. The dissolution profiles shown in Figs. 1 and 2 suggest that the formulation is similar to mono-component products - Diovan and Istin, which was also confirmed in an *in-vivo* bioequivalence study.

### Bioequivalence studies

The combinations of amlodipine/valsartan developed were tested *in-vivo,* in comparison to mono-component products - Istin® or Norvasc® for amlodipine besylate and Diovan® for valsartan.

**Table 6: Composition of strengths subjected to in-vivo testing**

| | **10/160 mg strength** | | **10/320 mg strength** | | **Function** |
|---|---|---|---|---|---|
| **Substance** | **Amount (mg)** | **Amount (% by weight)** | **Amount mg)** | **Amount (% by weight)** | |
| **Active ingredients** | | | | | |
| Valsartan | 160.000 | 36.782 | 320.000 | 41.667 | Active ingredient |
| Amlodipine besylate | 13.870 | 3.189 | 13.870 | 1.806 | Active ingredient |

| **Excipients in the core** | | | | | |
|---|---|---|---|---|---|
| Silicified microcrystalline cellulose | 67.000 | 15.402 | 134.000 | 17.448 | Filler |
| Sorbitol | 18.500 | 4.253 | 37.000 | 4.818 | Filler |
| Magnesium carbonate + pre-gelatinized starch | 18.500 | 4.253 | 37.000 | 4.818 | Filler |
| Pre-gelatinized starch | 6.000 | 1.379 | 12.000 | 1.563 | Filler |
| Povidone | 15.000 | 3.448 | 30.000 | 3.906 | Binder |
| Sodium stearyl fumarate | 9.000 | 2.069 | 17.000 | 2.214 | Lubricant |
| Sodium lauryl sulphate | 2.000 | 0.460 | 4.000 | 0.521 | Wetting agent |
| Crospovidone | 36.000 | 8.276 | 62.000 | 8.073 | Disintegrant |
| Colloidal silicon dioxide | 4.800 | 1.103 | 8.800 | 1.146 | Glidant |
| Microcrystalline cellulose | 74.330 | 17.087 | 74.330 | 9.678 | Filler |

| **Excipients in the coating** | | | | | |
|---|---|---|---|---|---|
| Hypromellose | 7.752 | 1.782 | 12.000 | 1.563 | Film-forming agent |
| Macrogol | 1.329 | 0.306 | 1.940 | 0.253 | Plasticizer |
| Titanium dioxide | 0.775 | 0.178 | 1.560 | 0.203 | Colorant |
| Talc | 0.111 | 0.026 | 2.300 | 0.299 | Glidant |
| Iron oxides | 0.033 | 0.008 | 0.200 | 0.026 | Colorant |

**Table 7: Results of bioequivalence studies**

| | **Amlodipine/valsartan 10/160 mg** | | **Amlodipine/valsartan 10/320 mg** | |
|---|---|---|---|---|
| | **Amlodipine** | **Valsartan** | **Amlodipine** | **Valsartan** |
| **AUC** | 100.9 % | 103.8 % | 98 % | 97.2 % |
| **cmax** | 98.6 % | 109.0 % | 100 % | 103.1 % |

**Example 5** - a mono-layer tablet prepared from a granulate containing valsartan (wet granulation), compacted amlodipine and powdered HCTZ

**Table 8**

| **Composition of 1 tbl.flm.** | **Prototype B (mg)** |
|---|---|
| Valsartan | 320.0 |
| Amlodipine besylate | 13.9 |
| Hydrochlorothiazide | 25.0 |
| Silicified microcrystalline cellulose | 134.0 |
| Sorbitol | 37.0 |
| Calcium carbonate + pre-gelatinized starch | 37.0 |
| Pre-gelatinized starch | 12.0 |
| Povidone | 305.0 |
| Sodium stearyl fumarate | 17.0 |
| Sodium lauryl sulphate | 4.0 |
| Crospovidone | 62.0 |
| Colloidal silicon dioxide | 8.8 |
| Microcrystalline cellulose | 74.3 |
| Coating layer (Macrogol, hypromellose, titanium dioxide, talc, iron | 30.0 |
| *Total* | *435.0* |

Valsartan, together with sorbitol, crospovidone, silicified microcrystalline cellulose, povidone and sodium lauryl sulfate is homogenized in a granulator and subsequently granulated by kneading with the use of water as a wetting agent. On achievement of the required granules the mixture is dried to the resulting granulate humidity of 1-3 to 3%. Amlodipine besylate, together with microcrystalline cellulose, crospovidone, colloidal silicon dioxide and sodium stearyl fumarate is screened through a sieve with the mesh size of 1.0 mm and the mixture is compacted in a compactor. The compacted material is admixed to the granulate together with the remaining excipients - the remaining part of crospovidone, pre-gelatinized starch, calcium carbonate with microcrystalline cellulose, sodium stearyl fumarate and colloidal silicon dioxide as well as the active ingredient hydrochlorothiazide. After final homogenization the tableting blend is compressed to solid pieces with a strength of at least 30 N. After the compression the cores are coated with a hypromellose suspension. When the weight of the cores has increased by 20 mg, the coating process is completed and the tablets are subjected to final drying. The temperature of the product should not exceed 42°C.

## Claims

1. A production method of a mono-layer tablet containing amlodipine and valsartan or their pharmaceutically acceptable salts, ***characterized in that***
a) valsartan or its pharmaceutically acceptable salt, together with at least one pharmaceutically acceptable excipient, is granulated with the use of water as a wetting agent,
b) amlodipine or its pharmaceutically acceptable salt, together with at least one pharmaceutically acceptable excipient, is dry granulated,
c) the obtained granules of amlodipine are mixed with the granules of valsartan and with at least one pharmaceutically acceptable excipient, the mixture is homogenized and the tableting blend is compressed to solid pieces - cores, and
d) the obtained cores are optionally provided with a coating.

2. The production method according to claim 1, ***characterized in that*** the mixture of valsartan and at least one pharmaceutically acceptable excipient according to item a) is wetted with water and the resulting mixture is processed into a granulate by kneading or fluidized granulation.

3. The production method according to claims 1 or 2, ***characterized in that*** at least one pharmaceutically acceptable excipient according to item a) is selected from the group comprising a filler, binder, wetting agent, disintegrant, or any combinations thereof.

4. The production method according to claim 1, ***characterized in that*** the dry mixture of amlodipine and at least one pharmaceutically acceptable excipient according to item b) is dry granulated by briquetting or compaction with the use of a force of 3 to 12 kN/cm.

5. The production method according to claims 1 to 4, ***characterized in that*** at least one pharmaceutically acceptable excipient according to item a) is selected from the group comprising a filler, disintegrant, glidant, lubricant or any combinations thereof.

6. The production method according to claims 1 to 5, ***characterized in that*** the granules of amlodipine are mixed with the granules of valsartan and at least one pharmaceutically acceptable excipient, the mixture is homogenized and the tableting blend is compressed to solid pieces having the minimum strength of 30 N.

7. The production method according to claims 1 to 6, ***characterized in that*** at least one pharmaceutically acceptable excipient according to item c) in the extragranular portion is selected from the group comprising a filler, disintegrant, glidant, lubricant or any combinations thereof.

8. The production method according to claims 1 to 7, ***characterized in that*** the filler is selected from the group comprising microcrystalline cellulose, powdered cellulose, calcium hydrogen phosphate, calcium carbonate, silicified microcrystalline cellulose, lactose monohydrate, anhydrous lactose, mannitol, sorbitol, lactitol, fructose, dextrans, sucrose, magnesium carbonate, starch, and pre-gelatinized starch.

9. The production method according to claims 1 to 7, ***characterized in that*** the binder is selected from the group comprising hydroxypropyl cellulose, hydroxyethyl cellulose, starch, pre-gelatinized starch, polymethacrylates, gelatine, hypromellose, povidone and microcrystalline cellulose

10. The production method according to claims 1 to 7, ***characterized in that*** the wetting agents is selected from the group comprising polysorbates and sodium lauryl sulfate.

11. The production method according to claims 1 to 7, ***characterized in that*** the disintegrant is selected from the group comprising crospovidone, croscarmellose sodium, starch, pre-gelatinized starch, microcrystalline cellulose, hydroxypropyl cellulose, sodium carboxymethyl starch, and polacrilin potassium.

12. The production method according to claims 1 to 7, ***characterized in that*** the glidant is selected from the group comprising talc, starch and colloidal silicon dioxide.

13. The production method according to claims 1 to 7, **characterized in that** the lubricant is selected from the group comprising magnesium stearate, stearic acid, magnesium silicate, calcium stearate, sodium stearyl fumarate, macrogols, hydrogenated vegetable oils, and sodium lauryl sulfate.

14. The production method of the mono-layer tablet according to claims 1 to 13, ***characterized in that*** hydrochlorothiazide is further added to the input mixture according to item c) containing granules of valsartan, granules of amlodipine and at least one pharmaceutically acceptable excipient.

15. The production method of the mono-layer tablet according to claim 14, ***characterized in that*** hydrochlorothiazide is added freely in the form of powder.

16. A mono-layer tablet, ***characterized in that*** it contains valsartan or a pharmaceutically acceptable thereof in an amount of 35 to 45% by weight in the form of granules prepared by wet granulation, amlodipine or a pharmaceutically acceptable salt thereof in an amount of 0.5 to 4% by weight in the form of granules prepared by dry granulation, a filler in an amount of 32 to 54.5% by weight, an antiadhesive agent in an amount of 0.2 to 2% by weight, a glidant in an amount of 0.1 to 1% by weight, a binder in an amount of 2 to 6% by weight, a disintegrant in an amount of 1 to 8% by weight and a wetting agent in an amount of 0.2 to 2% by weight.

17. The mono-layer tablet according to claim 16, ***characterized in that*** it further contains 5 to 50 mg of hydrochlorothiazide.

## Patentansprüche

1. Verfahren zur Herstellung einer Monoschichttablette, enthaltend Amlodipin und Valsartan oder deren pharmazeutisch annehmbare Salze, ***dadurch gekennzeichnet, dass***
a) Valsartan oder sein pharmazeutisch annehmbares Salz zusammen mit mindestens einem pharmazeutisch annehmbaren Hilfsstoff, unter Verwendung von Wasser als Benetzungsmittel, granuliert wird,
b) Amlodipin oder sein pharmazeutisch annehmbares Salz zusammen mit mindestens einem pharmazeutisch annehmbaren Hilfsstoff trocken granuliert wird,
c) das erhaltene Amlodipin-Granulat wird mit dem Valsartan-Granulat und mit mindestens einem pharmazeutisch annehmbaren Hilfsstoff vermischt, die Mischung wird homogenisiert und die Tablettiermischung wird zu festen Stücken - Kernen - gepresst, und
d) die erhaltenen Kerne werden gegebenenfalls mit einer Beschichtung versehen.

2. Herstellungsverfahren nach Anspruch 1, ***dadurch gekennzeichnet, dass*** das Gemisch aus Valsartan und mindestens einem pharmazeutisch annehmbaren Hilfsstoff gemäß Punkt a) mit Wasser benetzt wird und das resultierende Gemisch durch Kneten oder Wirbelschichtgranulierung zu einem Granulat verarbeitet wird.

3. Herstellungsverfahren nach Anspruch 1 oder 2, ***dadurch gekennzeichnet, dass*** mindestens ein pharmazeutisch annehmbarer Hilfsstoff gemäß Punkt a) aus der Gruppe ausgewählt ist, die einen Füllstoff, ein Bindemittel, ein Benetzungsmittel, ein Sprengmittel oder beliebige Kombinationen davon umfasst.

4. Herstellungsverfahren nach Anspruch 1, ***dadurch gekennzeichnet, dass*** die trockene Mischung aus Amlodipin und mindestens einem pharmazeutisch annehmbaren Hilfsstoff gemäß Punkt b) durch Brikettieren oder Kompaktieren, unter Anwendung einer Kraft von 3 bis 12 kN/cm, trocken granuliert wird.

5. Herstellungsverfahren nach den Ansprüchen 1 bis 4, ***dadurch gekennzeichnet, dass*** mindestens ein pharmazeutisch annehmbarer Hilfsstoff gemäß Punkt a) ausgewählt ist aus der Gruppe umfassend einen Füllstoff, ein Sprengmittel, Gleitmittel, Schmiermittel oder beliebige Kombinationen davon.

6. Herstellungsverfahren nach den Ansprüchen 1 bis 5, ***dadurch gekennzeichnet, dass*** das Amlodipin-Granulat mit dem Valsartan-Granulat und mindestens einem pharmazeutisch annehmbaren Hilfsstoff vermischt wird, die Mischung homogenisiert und die Tablettiermischung zu festen Stücken, welche eine Festigkeit von mindestens 30 N aufweisen, verpresst wird.

7. Herstellungsverfahren nach den Ansprüchen 1 bis 6, ***dadurch gekennzeichnet, dass*** mindestens ein pharmazeutisch annehmbarer Hilfsstoff gemäß Punkt c) im extragranulären Anteil aus der Gruppe ausgewählt ist, die einen Füllstoff, ein Sprengmittel, Gleitmittel, Schmiermittel oder beliebige Kombinationen davon umfasst.

8. Herstellungsverfahren nach den Ansprüchen 1 bis 7, ***dadurch gekennzeichnet, dass*** der Füllstoff ausgewählt ist aus der Gruppe umfassend mikrokristalline Cellulose, Cellulosepulver, Calciumhydrogenphosphat, Calciumcarbonat, verkieselte mikrokristalline Cellulose, Lactosemonohydrat, wasserfreie Lactose, Mannitol, Sorbitol, Lactitol, Fructose, Dextrane, Saccharose, Magnesiumcarbonat, Stärke und vorverkleisterte Stärke.

9. Herstellungsverfahren nach den Ansprüchen 1 bis 7, ***dadurch gekennzeichnet, dass*** das Bindemittel ausgewählt ist aus der Gruppe umfassend Hydroxypropylcellulose, Hydroxyethylcellulose, Stärke, vorverkleisterte Stärke, Polymethacrylate, Gelatine, Hypromellose, Povidon und mikrokristalline Cellulose.

10. Herstellungsverfahren nach den Ansprüchen 1 bis 7, ***dadurch gekennzeichnet, dass*** das Benetzungsmittel ausgewählt ist aus der Gruppe umfassend Polysorbate und Natriumlaurylsulfat.

11. Herstellungsverfahren nach den Ansprüchen 1 bis 7, ***dadurch gekennzeichnet, dass*** das Sprengmittel ausgewählt ist aus der Gruppe umfassend Crospovidon, Croscarmellose-Natrium, Stärke, vorverkleisterte Stärke, mikrokristalline Cellulose, Hydroxypropylcellulose, Natriumcarboxymethylstärke und Polacrilin-Kalium.

12. Herstellungsverfahren nach den Ansprüchen 1 bis 7, ***dadurch gekennzeichnet, dass*** das Gleitmittel ausgewählt ist aus der Gruppe umfassend Talkum, Stärke und kolloidales Siliciumdioxid.

13. Herstellungsverfahren nach den Ansprüchen 1 bis 7, ***dadurch gekennzeichnet, dass*** das Schmiermittel ausgewählt ist aus der Gruppe umfassend Magnesiumstearat, Stearinsäure, Magnesiumsilikat, Calciumstearat, Natriumstearylfumarat, Macrogole, hydrierte Pflanzenöle und Natriumlaurylsulfat.

14. Verfahren zur Herstellung einer Monoschichttablette nach den Ansprüchen 1 bis 13, ***dadurch gekennzeichnet, dass*** zusätzlich Hydrochlorothiazid zu der Eintragsmischung gemäß Punkt c), enthaltend Valsartan-Granulat, Amlodipin-Granulat und mindestens einen pharmazeutisch annehmbaren Hilfsstoff, zugegeben wird.

15. Verfahren zur Herstellung einer Einschichttablette nach Anspruch 14, ***dadurch gekennzeichnet, dass*** Hydrochlorothiazid frei in Form von Pulver zugegeben wird.

16. Einschichttablette, ***dadurch gekennzeichnet, dass*** sie Valsartan oder ein pharmazeutisch annehmbares Salz davon in einer Menge von 35 bis 45 Gew.-% in Form von Granulat, welches durch Feuchtgranulieren hergestellt wird, Amlodipin oder ein pharmazeutisch annehmbares Salz davon in einer Menge von 0,5 bis 4 Gew.-% in Form eines Granulats, das durch Trockengranulierung hergestellt wurde, einen Füllstoff in einer Menge von 32 bis 54,5 Gew.-%, ein Antihaftmittel in einer Menge von 0,2 bis 2 Gew.-%, ein Gleitmittel in einer Menge von 0,1 bis 1 Gew.-%, ein Bindemittel in einer Menge von 2 bis 6 Gew.-%, ein Sprengmittel in einer Menge von 1 bis 8 Gew.-% und ein Benetzungsmittel in einer Menge von 0,2 bis 2 Gew.-% enthält.

17. Monoschichttablette nach Anspruch 16, ***dadurch gekennzeichnet, dass*** sie weiterhin 5 bis 50 mg Hydrochlorothiazid enthält.

## Revendications

1. Procédé de production d'un comprimé monocouche contenant de l'amlodipine et du valsartan ou leurs sels pharmaceutiquement acceptables, **caractérisé en ce que**
a) le valsartan ou son sel pharmaceutiquement acceptable, associé à au moins un excipient pharmaceutiquement acceptable, est granulé en présence d'eau comme agent mouillant,
b) l'amlodipine ou son sel pharmaceutiquement acceptable, associé à au moins un excipient pharmaceutiquement acceptable, est granulé à sec,
c) les granules d'amlodipine obtenus sont mélangés avec les granules de valsartan et avec au moins un excipient pharmaceutiquement acceptable, le mélange étant homogénéisé et le mélange pour l'obtention de comprimés étant compressé en forme de morceaux solides-noyaux, et
d) les noyaux obtenus sont éventuellement pourvus d'un revêtement.

2. Le procédé de production selon la revendication 1, **caractérisé en ce que** le mélange de valsartan et d'au moins un excipient pharmaceutiquement acceptable selon le point a) est mouillé avec de l'eau et le mélange résultant est transformé en granulat par malaxage ou granulation en lit fluidisé.

3. Le procédé de production selon la revendication 1 ou 2, **caractérisé en ce qu'**au moins un excipient pharmaceutiquement acceptable selon le point a) est choisi dans le groupe comprenant une charge, un liant, un agent mouillant, un agent désintégrant ou toute combinaison de ceux-ci.

4. Le procédé de production selon la revendication 1, **caractérisé en ce que** le mélange sec d'amlodipine et d'au moins un excipient pharmaceutiquement acceptable selon le point b) est granulé à sec par briquetage ou compactage en utilisant une force de 3 à 12 kN / cm.

5. Le procédé de production selon les revendications 1 à 4, **caractérisé en ce qu'**au moins un excipient pharmaceutiquement acceptable selon le point a) est choisi dans le groupe comprenant une charge, un agent désintégrant, un agent de glissement, un lubrifiant ou toute combinaison de ceux-ci.

6. Le procédé de production selon les revendications 1 à 5, **caractérisé en ce que** les granules d'amlodipine sont mélangés avec les granules de valsartan et au moins un excipient pharmaceutiquement acceptable, le mélange est homogénéisé et le mélange pour l'obtention de comprimés est comprimé en morceaux solides ayant une résistance minimale de 30 N.

7. Le procédé de production selon les revendications 1 à 6, **caractérisé en ce qu'**au moins un excipient pharmaceutiquement acceptable selon le point c) dans la partie extragranulaire est choisi dans le groupe comprenant une charge, un agent désintégrant, un agent de glissement, un lubrifiant ou toute combinaison de ceux-ci.

8. Le procédé de production selon les revendications 1 à 7, **caractérisé en ce que** la charge est choisie dans le groupe comprenant : la cellulose microcristalline, la cellulose en poudre, l'hydrogénophosphate de calcium, le carbonate de calcium, la cellulose microcristalline silicifiée, le lactose monohydraté, le lactose anhydre, le mannitol, le sorbitol, le lactitol, le fructose, les dextranes, le saccharose, le carbonate de magnésium, l'amidon et l'amidon prégélatinisé.

9. Le procédé de production selon les revendications 1 à 7, **caractérisé en ce que** le liant est choisi dans le groupe comprenant l'hydroxypropylcellulose, l'hydroxyéthylcellulose, l'amidon, l'amidon prégélatinisé, les polyméthacrylates, la gélatine, l'hypromellose, la povidone et la cellulose microcristalline.

10. Le procédé de production selon les revendications 1 à 7, **caractérisé en ce que** les agents mouillants sont choisis dans le groupe comprenant les polysorbates et le laurylsulfate de sodium.

11. Le procédé de production selon les revendications 1 à 7, **caractérisé en ce que** l'agent désintégrant est choisi dans le groupe comprenant la crospovidone, la croscarmellose de sodium, l'amidon, l'amidon prégélatinisé, la cellulose microcristalline, l'hydroxypropylcellulose, l'amidon carboxyméthyle de sodium et la polacriline de potassium.

12. Le procédé de production selon les revendications 1 à 7, **caractérisé en ce que** l'agent de glissement est choisi dans le groupe comprenant le talc, l'amidon et le dioxyde de silicium colloïdal.

13. Le procédé de production selon les revendications 1 à 7, **caractérisé en ce que** le lubrifiant est choisi dans le groupe comprenant le stéarate de magnésium, l'acide stéarique, le silicate de magnésium, le stéarate de calcium, le stéarylfumarate de sodium, les macrogols, les huiles végétales hydrogénées et le laurylsulfate de sodium.

14. Le procédé de production du comprimé monocouche selon les revendications 1 à 13, **caractérisé en ce que** l'hydrochlorothiazide est en outre ajouté au mélange d'entrée selon le point c) contenant des granules de valsartan, des granules d'amlodipine et au moins un excipient pharmaceutiquement acceptable.

15. Le procédé de production du comprimé monocouche selon la revendication 14, **caractérisé en ce que** l'hydrochlorothiazide est ajouté librement sous forme de poudre.

16. Comprimé monocouche, **caractérisé en ce qu'**il contient du valsartan ou un de ses dérivés pharmaceutiquement acceptables en une quantité allant de 35 à 45% en poids sous forme de granules préparés par granulation par voie humide, de l'amlodipine ou un de ses sels pharmaceutiquement acceptables en une quantité allant de 0,5 à 4% en poids sous la forme de granules préparés par granulation à sec, une charge en une quantité allant de 32 à 54,5% en poids, un agent antiadhésif en une quantité allant de 0,2 à 2% en poids, un agent de glissement en une quantité allant de 0,1 à 1 % en poids, un liant en une quantité allant de 2 à 6% en poids, un agent désintégrant en une quantité allant de 1 à 8% en poids et un agent mouillant en une quantité allant de 0,2 à 2% en poids.

17. Le comprimé monocouche selon la revendication 16, **caractérisé en ce qu'**il contient en outre de 5 à 50 mg d'hydrochlorothiazide.
